# EUROPEAN PATENT SPECIFICATION

(11) **EP 4 255 984 B1**
(45) Date of publication and mention of the grant of the patent: **23.10.2024**
(21) Application number: 21816471.3
(22) Date of filing: 30.11.2021
(51) Int. Cl.: C08K 11/00, C08L 33/00, C08L 83/00, B33Y 10/00, B33Y 70/10, B33Y 80/00, A61L 27/00, B29C 64/00, C08G 77/00, C09D 183/04, A61L 27/16, A61L 27/18, A61L 27/36, C08G 77/04

(54) **USE OF RESIN-BASED BIOCOMPOSITE FOR 3D PRINTING IN A PROCESS FOR THE PREPARATION OF ORGAN OR TISSUE MODELS**
VERWENDUNG VON HARZBASIERTER BIOVERBUNDSTOFF FÜR 3D-DRUCK IN EINEM VERFAHREN ZUR HERSTELLUNG VON ORGAN- ODER GEWEBEMODELLEN
UTILISATION DE BIOCOMPOSITE À BASE DE RÉSINE POUR IMPRESSION 3D DANS UN PROCÉDÉ DE PRÉPARATION DE MODÈLES D'ORGANES OU DE TISSUS

(30) Priority: 01.12.2020 IT 202000029297
(43) Date of publication of application: 11.10.2023
(73) Proprietor: RIC3D S.r.l., 20158 Milan (IT); MTM S.r.l., 20158 Milan (IT); Techinnova Srl, 21059 Viggiu' (VA) (IT)
(72) Inventor: ROGGERI, Riccardo, 20159 Viggiu' (VA) (IT)
(74) Representative: Tagliafico, Giulia
(86) International application number: PCT/EP2021/083509
(87) International publication number: WO 2022/117544

(56) References cited:
- WO-A1-2004/083311
- WO-A1-2016/030409
- CN-A- 108 503 329
- JP-A- 2017 082 216

## Description

### TECNICAL FIELD OF THE INVENTION

The present invention relates to the use of a composition comprising one or more commercially available 3D printing resins mixed with plant-based material, in a process for the preparation of organ or tissue models, wherein such composition displays improved mechanical properties with respect to the corresponding unmixed resins in terms of flexibility, elasticity and prolonged use resistance, even in contact with physiological solutions.

Commercially available 3D printing resins are polymers suitable in stereolithographic printing (SLA) and Digital Light Processing (DLP), in particular acrylic resins (homo or co-polymers polyacrylates or mixtures thereof) and silicone resins (or rubbers).

The plant material derives from suitable treatment and grinding of agricultural waste, until particles of average size between 50 and 100 microns (mm) are obtained.

The invention also relates to the preparation of models (mock-ups) of organs or tissues using the composition of the invention, as well as to the models thus prepared.

### BACKGROUND OF THE INVENTION

It is known that printing technologies 3D have become an instrument widely used in many sectors, including the production of organ models, for example for teaching and/or simulating surgeries in preparation for particularly complex operations. This need has created an increasing demand for materials that allow to realize models with improved mechanical properties compared to those obtainable using the 3D printing resins existing on the market, in particular in terms of elasticity and flexibility.

The addition of plant fibres to the polymers can contribute to the improvement of the mechanical properties, even if it poses problems, such as the need for adequate drying of the natural, hydrophilic fibres, before the addition and the need to use polymers with low melting temperatures to avoid thermal degradation of the natural fibres themselves. The increasing attention to the environment, together with the need for new materials for rapid prototyping, have encouraged the search for compounds consisting of polymers and plant fillers.

In fact, additive polymers obtained by mixing synthetic resins with vegetable material are known.

Mazzanti, Malagutti, Mollica (Polymers 2019, 11, 1094) evaluate different polymers for 3D printing with fused deposition modelling (FDM) added with natural fillers (such as wood flour, rice or coconut husks, hemp fibres or flax) which, being mainly agricultural waste products often locally sourced, have the advantage of reducing costs and decreasing the environmental impact of the compound. The polymers studied are those typically used in FDM, such as polyethylene (PE), polypropylene (PP), acrylonitrile-butadiene-stirene (ABS), polycarbonate (PC), polysulfone (PSU), polyetherimide (PEI) and poly-(lactic acid) (PLA), of which it has been evaluated whether the addition of natural fibres can exert a reinforcing function, also by printing the composites with FDM technique instead of with traditional techniques (e.g. extrusion). The results show that ABS and PLA do not benefit from the addition of natural fillers, while other polymers such as polyolefins are more performing. No data, however, is reported on more elastic polymers, such as silicone rubbers or polynitrile rubbers for 3D printing and with devices different from those for FDM.

Mantia and Morreale (Composites: Part A 42 (2011) 579-588) illustrate the main research results on composite polymers with biodegradable fillers and from renewable sources. In particular, the production and characterization of polymeric composites based on recyclable polymers such as polyolefins, filled with fibres and particles extracted from plants (generally relatively abundant) or from plant wastes, is reported. The most known and used natural-organic fillers are flour and wood fibres. Other natural-organic fillers, including cellulose, cotton, flax, sisal, kenaf, jute, hemp, starch, began to find application.

Patent document IN05584CH2015 describes a biocomposite, in which the reinforcing material consists of vegetable fibers from scraps (pseudo-banana stem), which constitute a renewable source which is abundantly available. The polymer matrix is instead a propylene homo or copolymer in virgin or waste form. Properties such as tensile strength, elongation at break, impact strength and hardness of the resulting composite material show that the vegetable fillers used are durable with respect to the thermoplastic material as such. However, in this document no materials obtained from matrices other than PP are considered, nor vegetable wastes other than fibers derived from banana plants, which are not available in all geographical areas of the planet.

Patent document US2018127554 claims a biodegradable composite comprising one or more biodegradable polymers, selected from poly (butylene adipate-co-terephthalate) (PBAT), poly (lactic acid) (PLA), poly (butylene succinate) (PBS), poly (butylene succinate-co-adipate) (PBSA), polycaprolactone (PCL) and polyhydrosalcanoates (PHA), among others, including a filler present in an amount which can reach 50% by weight. The filler may include tea or coffee by-products, perennial grass, agricultural residue and the agricultural residue includes flakes, stems and leaves. An in situ method for producing the biodegradable composite is also claimed. However, such a document does not consider the process of preparing the natural material to be used as a filler, which is of importance for the final characteristics exhibited by the biocomposite.

Patent document US2013053476 claims a compound, comprising a coated reinforcing biofiber and a plastic resin, which once molded has a flexural modulus (ASTM D790) of at least 50% more than a compound in which the biofiber is not coated. In this compound the coated biofiber is composed of plastisol and 55-80% of biofiber. The plastic resin present in the compound is selected from the group consisting of acrylonitrile-butadiene-stirene (ABS), polyvinyl chloride (PVC), chlorinated polyvinyl chloride (CPVC), polybutylene terephthalate (PBT), polymethylmethacrylate (PMMA), stirene-acrylonitrile (San), polyphenylene ether (PPE), polycarbonate (PC), stirene-butadiene-stirene (SBS), acrylic polymers, polyolefins, polymethylmethacrylate (PMMA), polyethyleneterephthalate glycol comonomer (PETG), elastomeric thermoplastic copolyester (COPE) and combinations thereof, and the biofiber comprises vegetable material selected from the group consisting of wood fiber, wood flour, flax, grass fibrils, plant shell fragments, peels fragments, plant pulp, plant peel, seeds of plants, vegetable fibres and combinations thereof. In the claimed compound plastisol, when melted as resin, has a Shore D hardness (ASTM D2240-02 after 15 seconds) greater than 60, a tensile strength (ASTM D638) of more than about 48 MPa, a percent elongation (ASTM D638) of less than 10%. The document shows how rigid materials can be obtained by adding plastic polymeric resins with suitably treated natural vegetable fillers.

CN108503329A discloses compositions for 3D printing comprising a silicone resin and potato starch ether.

The prior art highlights the criticality of the choice of the polymeric component and of the choice and treatment of the natural vegetable filler, in order to obtain a biocomposite with particular characteristics of flexibility and elasticity.

In any case, no printing resin 3D currently available on the market allows to print (with any of the printing techniques 3D available) objects which have the mechanical characteristics required by the models of organs, for example the heart, such as to realistically represent the functions proper to that organ.

These characteristics are:
- high elasticity, or excellent ability to return to original shape and size after being subjected to deformation;
- good flexibility, understood as normally in mechanical physics, i.e. as inverse of stiffness;
- good "resistance", understood as the ability of the mock-up not to break once subjected to multiple fluid insertion and circulation cycles.

### DESCRIPTION OF THE INVENTION

The object of the present invention is to overcome the limitations of the prior art by providing a composite product based on polymers suitable for 3D SLA and/or DLP printing, with characteristics of elasticity and flexibility sufficient for the realization of organ models, in particular hearts, realistic for teaching and training of medical personnel or simulation of surgical operations and resisting multiple use cycles, in which the additional component derives from vegetable wastes.

The main object of the present invention is therefore the use of a composition comprising one or more commercially available 3D printing resins, selected from acrylic resins (homo- or co-polymers polyacrylates or mixtures thereof) and silicone resins (or rubbers), mixed with plant material selected from the families of Solenaceae (potatoes, Aubergines, tomatoes, peppers) and the genus Fragaria (strawberries) in a process for the preparation of organ or tissue models. The parts of the plants used are mainly leaves, roots and stems.

Another object of the present invention is the preparation of mock-ups of organs or tissues, for example humans, using the composition of the invention.

This preparation process comprises the following main steps:
1) printing the organ model using a commercially available 3D printing resin selected from acrylic resins (homo- or co-polymers polyacrylates or mixtures thereof) and silicone resins (or rubbers);
2) percolation on the model prepared in the previous step of the composition of the invention, comprising the same resin used for printing in the previous step mixed with material of plant origin selected in the families of Solenaceae (potatoes, aubergines, tomatoes, peppers) and in the genus Fragraria (strawberries).

A further object of the present invention are the products thus prepared. Such products can be models of organs or tissues.

The printing techniques 3D used in the first step preferably are
- SLA, stereolithographic printing, a technique based on the photopolymerization of a liquid resin, sensitive to ultraviolet radiation emitted by a laser beam (photopolymerization with consequent solidification of the resin subjected to the action of the laser beam); E
- DLP, digital light processing printing, is a type of tank polymerization which makes use of a liquid photopolymeric resin which, subjected to a light source, polymerizes solidifying (this is an evolution/variant of the SLA printing).

Other printing techniques, such as FDM, fused deposition modeling (which normally uses filament-shaped polymers), are not used with the resins of the invention, since the characteristics of the material are not suitable and clog the nozzle, thus irreparably damaging it.

Thermoplastic polyurethanes and PLA are not used as 3D printing resins, since:
- polyurethanes are not very suitable for the selected printing types and do not appear to impart an acceptable degree of elasticity and flexibility to the printed mock-ups; E
- PLA does not resist the circulation of liquids in hollow tubes created in the printed mock-up (simulating the blood vessels of the heart).

The process of the present invention allows to obtain a final product having physical characteristics similar to a product (even of medium size) Completely made of material having Shore A equal to or less than 20 and which maintains over time the flexibility and elasticity typical of materials with this Shore. At the same time, however, the final product undertakes a high degree of energy elongation and return and characteristics of impact or stress resistance, typical of materials with Shore A equal to or greater than 60 (wiper rubbers).

The molded parts of this material have the appearance and behave like the silicone molded parts, and are strong enough to be used for multiple cycles, even in critical dynamic stress situations, such as prolonged compressive stress, while maintaining their characteristics of elasticity, resolution and compactness over time, without cracks.

In the case of a mock-up (model) of a heart thus produced, it is therefore possible for example to attempt to simulate, with a discrete approximation (with suitable instrumentation and techniques) the phases of the cardiac cycle:
- isovolumetric contraction,
- ventricular ejection,
- isovolumetric relaxation, and
- auxotonic phase.

It is briefly noted here that the Shore scale is designed to test the hardness of elastomers or plastomers, for example rubber or plastic, characterized by reversible deformations. The elastic deformation of the material is measured in terms of the depth of penetration of a movable truncated cone point (Shore type A) which initially protrudes 2.5 mm from the hole of a plate and is subject to a constant force produced by a spring. By pressing the plate onto the surface of the material to be examined, the penetrator returns toward the inside of the hole of the plate itself in relation to the hardness. The Shore hardness is measured in a scale of from 0 to 100, proportional to the linear displacement of the tip, produced during the testing step on the elastic material, with respect to its initial rest position.

The ASTM D2240-00 method contemplates twelve different Shore measurement scales: Type A, B, C, D, DO, E, M, O, 00, 000, OOO-S, AND R. The difference between the scales is in the form/size of the penetrator and in the force applied thereto. In each of the scales the hardness may take values from 0 to 100. It is therefore always necessary to specify the hardness value together with the scale to which it refers, for example: hardness 80 Shore A, often referred to as 80A more briefly. The most used scales are in fact the A and D, respectively for materials of lower or higher hardness. Although the two scales have some overlap margin, there is no common conversion formula in their ranges, since the two hardnesses obtained with the type A and type D durometers exhibit a correlation strongly dependant on the type of material examined.

The material of vegetable origin, before being mixed with the printing resins 3D, is suitably treated. Preferably, the treatment of the vegetable material comprises the following steps:
a) washing and elimination of the earth (for example 30 minutes at room temperature in denatured water, by subsequent immersions in tanks);
b) ultrasonic bath in water (for example 15 minutes);
c) high-pressure blowing to completely eliminate impurities (repeated steps carried out for example for about 10 minutes);
d) first one-hour stowing with water vapor at 70-90°C, to eliminate bacteria;
e) second ventilated stuffing at 105°C for 5 hours or at 120°C for 20 minutes (for industrial production a fluid bed dryer with blown chains is used);
f) crushing (for example with blade crusher);
g) first grinding (for example with hammer mill);
h) second grinding (for example with a nail mill) to reach particle diameters of about 0.5 mm; and
i) micronization (for example with an elliptical chamber micronizer or with a mill with opposing jets) to reach below 100 micron and up to 50 micron.

Grinding takes place inside equipments such as grinding wheels (or mills), generally consisting of a grinding chamber inside which mobile grinding elements operate. These grinding elements exert a force on each particle to be ground; as a consequence, the particles can undergo an elastic (or reversible) deformation if, after the application of the force has ceased, they return to the original shape, or a plastic deformation, if they undergo an irreversible deformation. In addition to a certain applied force, for typical load values for each material, the particles are broken. Usually, the individual particles are crushed by following any irregularities or imperfections inside the individual particles.

The blade crusher is essentially formed by a grinding chamber, in which a horizontal shaft, to which blades are fixed, rotates. Below the chamber there is an interchangeable perforated grid. As the shaft rotates, the blades force the material against the grid by crushing it. All smaller pieces of the grid holes pass to an underlying collector (single pass).

The hammer mill consists of a cylindrical chamber and a central axis with 2-8 stainless steel articulated hammers. In the lower part of the mill there is an interchangeable grid which makes the ground material come out. When the axle rotates the hammers, due to the centrifugal force, they are arranged radially and grind the material introduced from above. This type of grinding exploits the impact forces and is suitable for grinding dry materials or fibrous materials, but also for wet grinding. In the case of hard and friable products, the particle size can reach up to 50 µm. In the case of the present invention, a single passage is realized, to reach particle diameters of about 0.5 mm.

The vertical elliptical chamber micronizing mill consists of a vertical elliptical chamber inside which the air or generally the gas enters under pressure (2-10 kg/cm²) through a series of nozzles located in the lower part. The material to be micronized enters by means of a feeder, is brought to high speed by the fluid and is ground; finally, the material in fine powder is made to leave the mill. With this process it is possible to reach about 75-100 microns.

The opposite jet mill operates in a similar manner to the previous one, with the fundamental difference that 2 opposing air jets enter the grinding chamber through two nozzles located on the same axis. Two jets collide in the micronization zone, causing the solid particles to collide, which thus break. The feeding takes place from a vibrating hopper, from which the already fine particles are entrained by the air jet. The micronization takes place by self-operation and friction. The particles are then transported into the classifying chamber; the smaller ones are dragged toward the outlet, the others fall into the zone and are re-launched into the micronization zone.

Some (non-limiting) examples of the present invention are given below.

### EXAMPLES

### Example 1

The first experimental tests were carried out in order to understand whether the component of vegetable origin could be added to commercial resins used for printing 3D to give the final molded product the characteristics of greater elasticity, flexibility and resistance to prolonged use.

The material of vegetable origin tested consists of fibers of vegetable origin deriving from the leaves, roots and stems of the following plants: lettuce, rocket, radicchio, beans, pumpkin, courgette, Solenacaee (tomato, aubergine, potato, peppers) and Fragraria (strawberries were selected). These were derived from scraps from aquaponic cultures.

The tests were carried out initially by preparing mixtures of plant components deriving from different plants.

The first tests were carried out using random mixtures of vegetable wastes, which had not been selected and divided. Subsequently it was decided to select (in Example 2) the individual plant varieties.

It was observed that this approach did not lead to significant improvements with respect to the characteristics of the starting 3D printing resins. First of all, in fact, the main problem was a different density coming from the original resins, combined with a higher density of the material of vegetable origin. The 50%-50% tested mixtures had always different densities. Probably due to the heterogeneous and always different mix of the vegetable sources used.

The experiment however was important, because it determined that a random mix of plant material does not serve to determine the change in elasticity condition, but it was necessary to select (as then best shown in Example 2) the individual plant varieties.

### Example 2

In a second experiment it was then decided to use the individual varieties of plants in the different parts (leaves, roots, drums) by varying the percentages of these individual parts inside the material of vegetable origin. Mixtures with a particle size of 100 microns were obtained.

Commercial 3D printing resins having a low hardness (lowest possible Shore A) have been selected for testing. In fact, the density of the resins is increased with lower Shore A, allowing to improve the general densitometry of the final compound.

Some results obtained using different plant components added to some of the most common 3D printing resins of the DLP and SLA type are reported below. In the former, UV sources are used to carry out the polymerization. Instead, laser sources are used in SLAs to obtain polymerization. These results are reported in Tables 1, 2 and 3.

**Table 1**

| **Material of plant origin** | **Resin % weight** | **Plant material%weight** | **Type of TEST** | **Flexibility result** | **Resistance Result** |
|---|---|---|---|---|---|
| Mix 50% of leaves and 50% of lettuce stems | 80 | 20 | Flexibility, resistance over time to stress | IN | IN |
| Mix 50% of leaves and 50% of lettuce stems | 70 | 30 | Flexibility, resistance over time to stress | NS | NS |
| Mix 50% of leaves and 50% of lettuce stems | 50 | 50 | Flexibility, resistance over time to stress | NS | NS |
| Lettuce leaves only | 80 | 20 | Flexibility, resistance over time to stress | IN | IN |
| Lettuce stems only | 80 | 20 | Flexibility, resistance over time to stress | IN | IN |
| Mix 50% of leaves and 50% of bean stems | 80 | 20 | Flexibility, resistance over time to stress | 2 | 2 |
| Mix 50% of leaves and 50% of bean stems | 70 | 30 | Flexibility, resistance over time to stress | NS | NS |
| Mix 50% of leaves and 50% of bean stems | 50 | 50 | Flexibility, resistance over time to stress | NS | NS |
| Only bean leaves | 80 | 20 | Flexibility, resistance over time to stress | 2 | 2 |
| Only bean stems | 80 | 20 | Flexibility, resistance over time to stress | IN | IN |
| Mix of 50% leaves and 50% courgette stems | 80 | 20 | Flexibility, resistance over time to stress | 3 | 3 |
| Mix of 50% leaves and 50% courgette stems | 70 | 30 | Flexibility, resistance over time to stress | IN | IN |
| Only courgette stems | 80 | 20 | Flexibility, resistance over time to stress | 3 | 3 |
| Only couregette leaves | 80 | 20 | Flexibility, resistance over time to stress | 3 | 3 |
| Mix 50% of leaves and 50% stems of Solenaceae | 80 | 20 | Flexibility, resistance over time to stress | 4 | 4 |
| Mix 50% of leaves and 50% stems of Solenaceae | 70 | 30 | Flexibility, resistance over time to stress | 4 | 4 |
| Only Solenaceae stems | 80 | 20 | Flexibility, resistance over time to stress | 3 | 4 |
| Only Solenaceae leaves | 80 | 20 | Flexibility, resistance over time to stress | 4 | 4 |
| Mix of 50% leaves and 50% Fragraria stems | 80 | 20 | Flexibility, resistance over time to stress | 4 | 4 |
| Mix of 50% leaves and 50% Fragraria stems | 70 | 30 | Flexibility, resistance over time to stress | 4 | 4 |
| Only Fragraria stems | 80 | 20 | Flexibility, resistance over time to stress | 3 | 4 |
| Only Fragraria leaves | 80 | 20 | Flexibility, resistance over time to stress | 4 | 4 |
| Mix 50% leaves, 40% stems and 10% Fragraria roots | 80 | 20 | Flexibility, resistance over time to stress | 4 | 5 |

| | | | | | |
|---|---|---|---|---|---|
| **Legend for all tables:** Test: F flexibility; R: Resistance to prolonged use and stresses flexibility index: 1 bad - 10 optimal; IN= unaltered; NS= No polymerization resistance index: 1 bad - 10 optimal; IN= unaltered; NS= No polymerization | | | | | |

### Index evaluation:

1-3 very little perceptible improvement;
4-5 little perceptible improvement;
6 perceptible but not evident improvement;
7-8 perceptible and quantifiable improvement to the naked eye with a +25% compared to the standard;
9 perceptible improvement and quantifiable in + 30% compared to the standard; and
10 perceptible and quantifiable improvement over 30% compared to the standard.

**Table 2 - Experiments with Resin for 3D printing = RESIN DLP SHORE A 7**

| **Material of plant origin** | **Resin% weight** | **Plant material % weight** | **Type of TEST** | **Flexibility result** | **Resistance Result** | **Notes** |
|---|---|---|---|---|---|---|
| Mix 50% of leaves and 50% of lettuce stems | 80 | 20 | Flexibility, resistance over time to stress | 2 | 2 | Polymerization times increased by 25% |
| Mix 50% of leaves and 50% of lettuce stems | 70 | 30 | Flexibility, resistance over time to stress | NS | NS | |
| Mix 50% of leaves and 50% of lettuce stems | 50 | 50 | Flexibility, resistance over time to stress | NS | NS | |
| Lettuce leaves only | 80 | 20 | Flexibility, resistance over time to stress | IN | IN | Polymerization times increased by 25% |
| Lettuce stems only | 80 | 20 | Flexibility, resistance over time to stress | 2 | 2 | Polymerization times increased by 25% |
| Mix 50% of leaves and 50% of bean stems | 80 | 20 | Flexibility, resistance over time to stress | 2 | 2 | Polymerization times increased by 25% |
| Mix 50% of leaves and 50% of bean stems | 70 | 30 | Flexibility, resistance over time to stress | NS | NS | |
| Mix 50% of leaves and 50% of bean stems | 50 | 50 | Flexibility, resistance over time to stress | NS | NS | |
| Only bean leaves | 80 | 20 | Flexibility, resistance over time to stress | IN | IN | Polymerization times increased by 25% |
| Only bean stems | 80 | 20 | Flexibility, resistance over time to stress | IN | IN | Polymerization times increased by 25% |
| Mix of 50% leaves and 50% courgette stems | 80 | 20 | Flexibility, resistance over time to stress | IN | IN | Polymerization times increased by 25% |
| Mix of 50% leaves and 50% courgette stems | 70 | 30 | Flexibility, resistance over time to stress | 4 | 4 | Polymerization times increased by 25% |
| Only courgette stems | 80 | 20 | Flexibility, resistance over time to stress | IN | 4 | Polymerization times increased by 25% |
| Only couregette leaves | 80 | 20 | Flexibility, resistance over time to stress | IN | IN | Polymerization times increased by 25% |
| Mix 50% of leaves and 50% stems of Solenaceae | 80 | 20 | Flexibility, resistance over time to stress | IN | 4 | Polymerization times increased by 25% |
| Mix 50% of leaves and 50% stems of Solenaceae | 70 | 30 | Flexibility, resistance over time to stress | 4 | 5 | Polymerization times increased by 25% |
| Only Solenaceae stems | 80 | 20 | Flexibility, resistance over time to stress | 3 | 4 | Polymerization times increased by 25% |
| Only Solenaceae leaves | 80 | 20 | Flexibility, resistance over time to stress | 4 | 4 | Polymerization times increased by 25% |
| Mix of 50% leaves and 50% Solenaceae stems | 60 | 40 | Flexibility, resistance over time to stress | 5 | 5 | Polymerization times increased by 25% |
| Mix 50% of leaves and 40% stems of Solenaceae 10% roots | 60 | 40 | Flexibility, resistance over time to stress | 5 | 5 | Polymerization times increased by 25% |
| Mix of 50% leaves and 50% Fragraria stems | 80 | 20 | Flexibility, resistance over time to stress | 4 | 4 | Polymerization times increased by 25% |
| Mix of 50% leaves and 50% Fragraria stems | 70 | 30 | Flexibility, resistance over time to stress | 5 | 4 | Polymerization times increased by 25% |
| Only Fragraria stems | 80 | 20 | Flexibility, resistance over time to stress | 4 | 5 | Polymerization times increased by 25% |
| Only Fragraria leaves | 80 | 20 | Flexibility, resistance over time to stress | 5 | 4 | Polymerization times increased by 25% |
| Mix 50% leaves, 40% stems and 10% Fragraria roots | 70 | 30 | Flexibility, resistance over time to stress | 5 | 5 | Polymerization times increased by 25% |

**Table 3 - Experiments with Resin for 3D printing = RESIN DLP or SLA SHORE A 60**

| **Commercial name** | **Standard Flexibility Index** | **Material of plant origin** | **Resin% weight** | **Plant material % weight** | **Type of TEST** | **Flexibility result** | **Resistance result** | **Notes** |
|---|---|---|---|---|---|---|---|---|
| WanHao Clear Resin. Fonnalabs Clear Resin | 4 | Mix 50% of leaves and 50% of lettuce stems | 80 | 20 | Flexibility, resistance over time to stress | 2 | 2 | Polymerization times increased by 33% |
| WanHao Clear Resin. Fonnalabs Clear Resin | 4 | Mix 50% of leaves and 50% of lettuce stems | 70 | 30 | Flexibility, resistance over time to stress | 2 | IN | Polymerization times increased by 33% |
| WanHao Clear Resin. Fonnalabs Clear Resin | 4 | Mix 50% of leaves and 50% of lettuce stems | 50 | 50 | Flexibility, resistance over time to stress | NS | NS | |
| WanHao Clear Resin. Fonnalabs Clear Resin | 4 | Lettuce leaves only | 80 | 20 | Flexibility, resistance over time to stress | 2 | 2 | Polymerization times increased by 33% |
| WanHao Clear Resin. Fonnalabs Clear Resin | 4 | Lettuce stems only | 80 | 20 | Flexibility, resistance over time to stress | 2 | 2 | Polymerization times increased by 33% |
| WanHao Clear Resin. Fonnalabs Clear Resin | 4 | Mix 50% of leaves and 50% of bean stems | 80 | 20 | Flexibility, resistance over time to stress | 3 | 3 | Polymerization times increased by 33% |
| WanHao Clear Resin. Fonnalabs Clear Resin | 4 | Mix 50% of leaves and 50% of bean stems | 70 | 30 | Flexibility, resistance over time to stress | 3 | 3 | |
| WanHao Clear Resin. Fonnalabs Clear Resin | 4 | Mix 50% of leaves and 50% of bean stems | 50 | 50 | Flexibility, resistance over time to stress | NS | NS | |
| WanHao Clear Resin. Formalabs Clear Resin | 4 | Only bean leaves | 80 | 20 | Flexibility, resistance over time to stress | 3 | IN | Polymerization times increased by 33% |
| WanHao Clear Resin. Formalabs Clear Resin | 4 | Only bean stems | 80 | 20 | Flexibility, resistance over time to stress | IN | 3 | Polymerization times increased by 33% |
| WanHao Clear Resin. Formalabs Clear Resin | 4 | Mix of 50% leaves and 50% courgette stems | 80 | 20 | Flexibility, resistance over time to stress | IN | IN | Polymerization times increased by 33% |
| WanHao Clear Resin. Fonnalabs Clear Resin | 4 | Mix of 50% leaves and 50% courgette stems | 70 | 30 | Flexibility, resistance over time to stress | 4 | 5 | Polymerization times increased by 33% |
| WanHao Clear Resin. Fonnalabs Clear Resin | 4 | Only courgette stems | 80 | 20 | Flexibility, resistance over time to stress | 5 | IN | Polymerization times increased by 33% |
| WanHao Clear Resin. Fonnalabs Clear Resin | 4 | Only couregette leaves | 80 | 20 | Flexibility, resistance over time to stress | IN | IN | Polymerization times increased by 33% |
| WanHao Clear Resin. Fonnalabs Clear Resin | 4 | Mix 50% of leaves and 50% stems of Solenaceae | 80 | 20 | Flexibility, resistance over time to stress | IN | 5 | Polymerization times increased by 33% |
| WanHao Clear Resin. Fonnalabs Clear Resin | 4 | Mix 50% of leaves and 50% stems of Solenaceae | 70 | 30 | Flexibility, resistance over time to stress | 5 | 5 | Polymerization times increased by 33% |
| WanHao Clear Resin. Fonnalabs Clear Resin | 4 | Only Solenaceae stalks | 80 | 20 | Flexibility, resistance over time to stress | 3 | 5 | Polymerization times increased by 33% |
| WanHao Clear Resin. Fonnalabs Clear Resin | 4 | Only Solenaceae leaves | 80 | 20 | Flexibility, resistance over time to stress | 5 | IN | Polymerization times increased by 33% |
| WanHao Clear Resin. Fonnalabs Clear Resin | 4 | Mix of 50% leaves and 50% Solenaceae stems | 60 | 40 | Flexibility, resistance over time to stress | IN | 6 | Polymerization times increased by 33% |
| WanHao Clear Resin. Formalabs Clear Resin | 4 | Mix 50% of leaves and 40% stems of Solenaceae 10% roots | 60 | 40 | Flexibility, resistance over time to stress | 6 | 5 | Polymerization times increased by 33% |
| WanHao Clear Resin. Formalabs Clear Resin | 4 | Mix of 50% leaves and 50% Fragraria stems | 80 | 20 | Flexibility, resistance over time to stress | 4 | IN | Polymerization times increased by 33% |
| WanHao Clear Resin. Fonnalabs Clear Resin | 4 | Mix of 50% leaves and 50% Fragraria stems | 70 | 30 | Flexibility, resistance over time to stress | 5 | IN | Polymerization times increased by 33% |
| WanHao Clear Resin. Fonnalabs Clear Resin | 4 | Only Fragraria stems | 80 | 20 | Flexibility, resistance over time to stress | IN | 5 | Polymerization times increased by 33% |
| WanHao Clear Resin. Fonnalabs Clear Resin | 4 | Only Fragraria leaves | 80 | 20 | Flexibility, resistance over time to stress | 5 | IN | Polymerization times increased by 33% |
| WanHao Clear Resin. Fonnalabs Clear Resin | 4 | Mix 50% leaves, 40% stems and 10% Fragraria roots | 70 | 30 | Flexibility, resistance over time to stress | 6 | 5 | Polymerization times increased by 33% |
| WanHao Clear Resin. Formalabs Clear Resin | 4 | Mix 50% of stems, leaves and roots of Solenaceae and 50% of stems, leaves and roots of Fragaria | 70 | 30 | Flexibility, resistance over time to stress | 6 | 5 | Polymerization times increased by 35% |
| WanHao Clear Resin. Fonnalabs Clear Resin | 4 | Mix 50% of stems, leaves and roots of Solenaceae and 50% of stems, leaves and roots of Fragaria | 65 | 35 | Flexibility, resistance over time to stress | 6 | 6 | Polymerization times increased by 35% |
| WanHao Clear Resin. Fonnalabs Clear Resin | 4 | Mix 50% of stems, leaves and roots of Solenaceae and 50% of stems, leaves and roots of Fragaria | 60 | 40 | Flexibility, resistance over time to stress | 6 | 6 | Polymerization times increased by 35% |

This experiment was useful not only to better understand which plant products were the best to use (Solenaceae and Fragraria), but it showed that the best plant material is that composed of: 50% leaves, 40% stems and 10% roots.

### Example 3

In a third experiment only those deriving from Solenaceae and Fragraria were used as components of plant origin, in particular with derivatives of tomato, pepper and strawberry plants.

Moreover, the process of obtaining the material of vegetable origin and its micronization was improved, reaching a product with particle size below 100 microns. This result has led to a marked improvement in the component of plant origin, which has led to better results in terms of strength and elasticity.

A process was then carried out comprising the following steps:
a) washing and elimination of the earth (30 minutes at room temperature in denatured water, by subsequent immersions in tanks);
b) ultrasonic bath in water (15 minutes);
c) high-pressure blowing to completely eliminate impurities (repeated steps carried out for example for about 10 minutes);
d) first one-hour stowing with water vapor at 70-90°C, to eliminate bacteria;
e) second ventilated plastering at 105°C for 5 hours or at 120°C for 20 minutes (for industrial production a fluid bed drier with blown chains is used);
f) crushing (with blade crusher);
g) first grinding (with hammer mill);
h) second grinding (with nail mill) to reach particle diameters of about 0.5 mm; and
i) micronization (with an elliptical chamber micronizer or with a Molino with opposite jets) to reach below 100 micron and up to 50 micron.

Once micronized, the material is added to the printing resin 3D, at predetermined concentrations, as reported in Tables 4 and 5.

The results obtained with mixtures containing components of vegetable origin at 15%, 20%, 25%, 30%, 40% and 50% (by weight) are reported below.

It is recalled that the plant material was composed of: 50% Solenaceae (50% leaves, 40% stems, 10% roots) and 50% Fragraria (50% leaves, 40% stems), 10% roots).

**Table 4**

| **Resin for 3D printing** | **Commercial name** | **Standard Flexibility Index** | **Material of plant origin** | **Resin % weight** | **Plant material % weight** | **Type of TEST** | **Flexibility result** | **Resistance result** | **Notes** |
|---|---|---|---|---|---|---|---|---|---|
| | WanHao Clear Resin. Formalabs Clear Resin | 4 | Mix 50% of stems, leaves and roots of solenaceae and 50% of stems, leaves and roots of Fragaria | 50 | 50 | Flexibility, resistance over time to stress | 6 | 5 | Polyme rization times increas ed by 33% |
| RESIN DLP or SLA SHORE A 65 | WanHao Clear Resin. Formalabs Clear Resin | 4 | Mix 50% of stems, leaves and roots of solenaceae and 50% of stems, leaves and roots of Fragaria | 60 | 40 | Flexibility, resistance over time to stress | 7 | 6 | Polyme rization times increas ed by 35% |
| | WanHao Clear Resin. Formalabs Clear Resin | 4 | Mix 50% of stems, leaves and roots of solenaceae and 50% of stems, leaves and roots of Fragaria | 70 | 30 | Flexibility, resistance over time to stress | 7 | 7 | Polyme rization times increas ed by 35% |
| | WanHao Clear Resin. Formalabs Clear Resin | 4 | Mix 50% of stems, leaves and roots of solenaceae and 50% of stems, leaves and roots of Fragaria | 75 | 25 | Flexibility, resistance over time to stress | 7 | 7 | Polyme rization times increas ed by 30% |
| | WanHao Clear Resin. Formalabs Clear Resin | 4 | Mix 50% of stems, leaves and roots of solenaceae and 50% of stems, leaves and roots of Fragaria | 80 | 20 | Flexibility, resistance over time to stress | 6 | 7 | Polyme rization times increas ed by 30% |
| | WanHao Clear Resin. Formalabs Clear Resin | 4 | Mix 50% of stems, leaves and roots of solenaceae and 50% of stems, leaves and roots of Fragaria | 85 | 15 | Flexibility, resistance over time to stress | 6 | 6 | Polyme rization times increas ed by 30 % |

**Table 5**

| **Resin for 3D printing** | **Commercial name** | **Standard Flexibility Index** | **Material of plant origin** | **Resin % weight** | **Plant material % weight** | **Type of TEST** | **Flexibility result** | **Resistance result** | **Notes** |
|---|---|---|---|---|---|---|---|---|---|
| | WanHao Clear Resin. Fonnalabs Clear Resin | 4 | Mix 50% of stems, leaves and roots of solenaceae and 50% of stems, leaves and roots of Fragaria | 50 | 50 | Flexibility, resistance over time to stress | 6 | 6 | Polymeriz ation times increased by 33% |
| RESIN DLP or SLA SHORE A 60 | WanHao Clear Resin. Fonnalabs Clear Resin | 4 | Mix 50% of stems, leaves and roots of solenaceae and 50% of stems, leaves and roots of Fragaria | 60 | 40 | Flexibility, resistance over time to stress | 7 | 6 | Polymeriz ation times increased by 35% |
| | WanHao Clear Resin. Fonnalabs Clear Resin | 4 | Mix 50% of stems, leaves and roots of solenaceae and 50% of stems, leaves and roots of Fragaria | 70 | 30 | Flexibility, resistance over time to stress | 7 | 7 | Polymeriz ation times increased by 35% |
| | WanHao Clear Resin. Fonnalabs Clear Resin | 4 | Mix 50% of stems, leaves and roots of solenaceae and 50% of stems, leaves and roots of Fragaria | 75 | 25 | Flexibility, resistance over time to stress | 8 | 7 | Polymeriz ation times increased by 30% |
| | WanHao Clear Resin. Fonnalabs Clear Resin | 4 | Mix 50% of stems, leaves and roots of solenaceae and 50% of stems, leaves and roots of Fragaria | 80 | 20 | Flexibility, resistance over time to stress | 8 | 8 | Polymeriz ation times increased by 30% |
| | WanHao Clear Resin. Fonnalabs Clear Resin | 4 | Mix 50% of stems, leaves and roots of solenaceae and 50% of stems, leaves and roots of Fragaria | 85 | 15 | Flexibility, resistance over time to stress | 7 | 8 | Polymeriz ation times increased by 30% |

Although under the best conditions (SLA resin with SHORE A 60) and material of vegetable origin at 20% it was found that the resistance and elasticity properties conferred to the realized mockups, they retained their characteristics for no more than 3 days and then gradually lost their elasticity, reaching values similar to the standard resin as such, after about 7 days of exposure to light and air.

In the course of this third test set, it has been recognized, in a random manner, that by percolating the composition of the invention over a structure (racks) made of the standard resin as such, the elasticity properties were stored for more than 15 days.

### Example 4

In this experiment, shelves made with a honeycomb shape of about 300 microns in diameter with the 3D standard SHORE A 60 printing resin were used, on which a composition, composed of silicone rubber (distributed by Resin Srl of La Spezia) is percolated with a Shore A 20 or Shore A 15. These silicone rubbers must be activated with a suitable catalyst containing dimethylbis((1-oxoneodecyl)oxy)stannane, ethyl silicate, ethyl polysilicate.

The scaffold thus produced was then left in the laminar chamber sucked, under UV lamp, for about 10 minutes.

In this case, mixtures were carried out with different percentages of silicone rubber, catalyst and material of vegetable origin.

The main characteristic of this compound is to polymerize directly, without the need for UV or laser sources, given the presence of a catalyzing agent acting at room temperature. The results obtained with such compositions are reported in Table 6.

**Table 6 - Experiments made with 3D printed shelves using RESIN SLA SHORE A 60 (trade name: WanHao Clear Resin. Formalabs Clear Resin) - Standard flexibility index: 4**

| **Resin for 3D printing** | **Catalyst** | **Material of plant origin** | **Resin% weight** | **Catalyst % weight** | **Plant material % weight** | **Type of TEST** | **Flexibility result** | **Resistance result** | **Notes** |
|---|---|---|---|---|---|---|---|---|---|
| Resin Srl Shore A 20 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 80 | 5 | 15 | Flexibility, resistance over time to stress | 7 | 7 | Polymerization times 50 minutes under a lamellar hood |
| Resin Srl Shore A 20 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 75 | 5 | 20 | Flexibility, resistance over time to stress | 7 | 8 | Polymerization times 50 minutes under a lamellar hood |
| Resin Srl Shore A 20 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 70 | 5 | 25 | Flexibility, resistance over time to stress | 8 | 8 | Polymerization times 50 minutes under a lamellar hood |
| Resin Srl Shore A 20 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 65 | 10 | 25 | Flexibility, resistance over time to stress | 8 | 9 | Polymerization times 40 minutes under a lamellar hood |
| Resin Srl Shore A 15 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 80 | 5 | 15 | Flexibility, resistance over time to stress | 7 | 7 | Polymerization times 50 minutes under a lamellar hood |
| Resin Srl Shore A 15 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 75 | 5 | 20 | Flexibility, resistance over time to stress | 8 | 8 | Polymerization times 50 minutes under a lamellar hood |
| Resin Srl Shore A 15 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 70 | 5 | 25 | Flexibility, resistance over time to stress | 8 | 8 | Polymerization times 50 minutes under a lamellar hood |
| Resin Srl Shore A 15 silicone rubber | dimethylbis ((1-oxoneodecyl) oxy) stannan, ethyl silicate, ethyl poly silicate | Mix 50% of stems, leaves and roots of solenaceae and 50% of stems, leaves and roots of Fragaria | 65 | 10 | 25 | Flexibility, resistance over time to stress | 9 | 9 | Polymerization times 35minutes under a lamellar hood |

In the best conditions (3D molded shelves with SLA SHORE 60 resin and percolated with 65% SHORE A 15 silicone rubber, 25% vegetable material and 10% catalyst) the elasticity and strength capacities are improved by 50% compared to a normal mock-ups molded with SLA SHORE a 60 resin. In practice, it is as if the mock-up had a elasticity with SHORE a 40, while maintaining a better resistance and wear over time than the standard one.

Moreover, these characteristics are maintained for 3 weeks, after which a loss of elasticity equal to 5% occurs.

### Example 5

In this experiment an SLA resin of the acrylic type was used with SHORE A equal to 50 (elastic resin SHORE 50 of Formlabs), which has the following characteristics: SHORE A hardness: 50; elongation at break % 160; tensile strength (MPa) 3.2; tear strength (kn/m) 19.1.

The experiments were carried out in order to be able to realize the honeycomb shelving with different types of printers (Formlabs, Wanhao, Anycubic), reducing the honeycomb structure to 250 microns.

Tests were then carried out using various mixtures having the following compositions:
- 65% Shore A 15 silicone rubber, 25% plant component, 10% catalyst
- 75% SHORE A acrylic resin, 25% plant component, With application of UV source
- SHORE A 50 40% acrylic resin, 30% silicone rubber plant component 25%, 5% catalyst with application of UV source
- SHORE A 50 40% acrylic resin, 25% silicone rubber plant component 25%, Catalyst 10% with application of UV source

The results obtained are further improved and are reported in the following Table 7.

**Table 7 - Experiments made with 3D printed shelves using Elastic Resin Shore 50 (trade name: Formalabs) - Standard flexibility index: 6**

| **Resin for 3D printing 1** | **Resin for 3D printing 2** | **Catalyst** | **Material of plant origin** | **Resin 1 % weight** | **Resin 2% weight** | **Catalyst % weight** | **Plant material % weight** | **Type of TEST** | **Flexibility result** | **Resistance result** | **Notes** |
|---|---|---|---|---|---|---|---|---|---|---|---|
| Resin Srl Shore A 15 silicone rubber | no | dimethylb is ((1-oxoneode cyl) oxy) stannan, ethyl silicate, ethyl poly silica te | Mix 50% of stems, leaves and roots of solenaceae and 50% of stems, leaves and roots of Fragaria | 65 | 0 | 10 | 25 | Flexibility, resistance over time to stress | 9 | 9 | Polym erizatio n times 30 minute s under a lamella r hood |
| Elastic Resin Shore 50 | no | dimethylb is ((1-oxoneode cyl) oxy) stannan, ethyl silicate, ethyl poly silica te | Mix 50% of stems, leaves and roots of solenaceae and 50% of stems, leaves and roots of Fragaria | 75 | 0 | 0 | 25 | Flexibility, resistance over time to stress | 8 | 9 | Polym erizatio n times 50 minute s under a lamella r hood |
| Elastic Resin Shore 50 | Resin Srl Shore A 15 silicone rubber | dimethylb is ((1-oxoneode cyl) oxy) stannan, ethyl silicate, ethyl poly silica te | Mix 50% of stems, leaves and roots of solenaceae and 50% of stems, leaves and roots of Fragaria | 40 | 30 | 5 | 25 | Flexibility, resistance over time to stress | 9 | 10 | Polym erizatio n times 30 minute s under a lamella r hood |
| Elastic Resin Shore 50 | Resin Srl Shore A 15 silicone rubber | dimethylb is ((1-oxoneode cyl) oxy) stannan, ethyl silicate, ethyl poly silica te | Mix 50% of stems, leaves and roots of solenaceae and 50% of stems, leaves and roots of Fragaria | 40 | 25 | 10 | 25 | Flexibility, resistance over time to stress | 10 | 10 | Polym erizatio n times 25 minute s under a lamella r hood |

It is noted that, under the best conditions obtainable, the obtained mock-up maintains a 65% elasticity higher than the printing 3D as such, i.e. as if it had an elasticity and flexibility of a molded article with a hypothetical SHORE a 20 resin, but with a capacity not to break once subjected to several cycles of insertion and circulation of fluids equal to a mock-up made with resins at SHORE A 60.

These conditions were maintained for at least 4 weeks.

## Claims

1. Use of a composition comprising one or more commercially available resins for 3D printing, selected from the acrylic resins, optionally homo- or co-polymers polyacrylates or their mixtures thereof, and silicone resins, mixed with material of plant origin selected in the families of the Solenaceae, optionally potatoes, eggplant, tomatoes, peppers and in the genus of Fragaria, optionally strawberries, in a process for the preparation of organ or tissue models.

2. The use according to claim 1, wherein the plant parts, from which the plant material derives, are leaves, roots or stems or mixtures thereof.

3. The use according to any one of claims 1 or 2, wherein the material of plant origin derives from agricultural waste.

4. The use according to any one of claims 1 or 3, wherein the material of plant origin presents itself in the form of particles having an average size between 50 and 100 microns.

5. The use according to claim 1, wherein the process comprises the following steps:
1) printing an organ or tissue model using a resin commercially available for 3D printing selected from the acrylic resins, optionally homo- or co-polymers or their mixtures thereof, and silicone resins;
2) percolating on the model prepared in the previous step of the composition defined in claim 1, comprising the same resin used for printing in the previous step.

6. A process for preparing a 3D printed object, wherein the 3D printed object is an organ or tissue model, comprising the following steps:
1) printing the object using a resin commercially available for 3D printing selected from the acrylic resins, optionally homo- or co-polymers or their mixtures thereof, and silicone resins;
2) percolating on the object, prepared in the previous step, the composition defined in claim 1, comprising the same resin used for printing in the previous step.

7. The process according to claim 6, comprising the following pre-treatment of the material of plant origin:
a) washing and soil removal, optionally 30 minutes at room temperature in denatured water, by subsequent immersions in tanks; 30
b) ultrasonic bathing in water, optionally for 15 minutes; 2
c) blowing at high pressure, in order to completely eliminate the impurities, optionally in repeated steps carried out for about 10 minutes;
d) first stewing for one hour with water steam at 70-90°C, to eliminate the bacteria;
e) second stewing at 105°C for 5 hours with ventilation or at 120° for 20 minutes or with a fluid bed dryer with insufflate catenaries; 5
f) crushing, optionally with a blade crusher;
g) first grinding, optionally with a hammer mill;
h) second grinding, optionally with a nail mill, to reach particle diameters of about 0.5 mm; and
i) micronizing, optionally with a micronizer with an elliptical chamber or with a mill 10 having opposing jets, to reach below 100 microns and up to 50 microns.

8. A product obtained by the process according to any one of claims 6 or 7.

9. The product according to claim 8, in which the organ is a heart.

## Patentansprüche

1. Verwendung einer Zusammensetzung, die ein oder mehrere handelsübliche Harze für den 3D-Druck, ausgewählt aus Acrylharzen, gegebenenfalls Homo- oder Copolymeren von Polyacrylaten oder deren Mischungen, und Silikonharzen umfasst, gemischt mit Material pflanzlichen Ursprungs, ausgewählt aus den Familien der Solenaceae, gegebenenfalls Kartoffeln, Auberginen, Tomaten, Paprika und aus der Gattung Fragaria, gegebenenfalls Erdbeeren, in einem Verfahren zur Herstellung von Organ- oder Gewebemodellen.

2. Verwendung nach Anspruch 1, wobei die Pflanzenteile, von denen das Pflanzenmaterial stammt, Blätter, Wurzeln oder Stämme oder Mischungen davon sind.

3. Verwendung nach einem der Ansprüche 1 oder 2, wobei das Material pflanzlichen Ursprungs aus landwirtschaftlichen Abfällen stammt.

4. Verwendung nach einem der Ansprüche 1 oder 3, wobei das Material pflanzlichen Ursprungs in Form von Partikeln mit einer durchschnittlichen Größe zwischen 50 und 100 Mikron vorliegt.

5. Verwendung nach Anspruch 1, wobei das Verfahren die folgenden Schritte umfasst:
(1) Drucken eines Organ- oder Gewebemodells unter Verwendung eines im Handel erhältlichen Harzes für den 3D-Druck, ausgewählt aus Acrylharzen, gegebenenfalls Homo- oder Co-Polymeren oder deren Mischungen, und Silikonharzen;
(2) Perkolieren des im vorhergehenden Schritt hergestellten Modells mit der in Anspruch 1 definierten Zusammensetzung, die das gleiche Harz umfasst, das für den Druck im vorhergehenden Schritt verwendet wurde.

6. Verfahren zur Herstellung eines 3D-gedruckten Objekts, wobei das 3D-gedruckte Objekt ein Organ- oder Gewebemodell ist, umfassend die folgenden Schritte:
(1) Drucken des Objekts unter Verwendung eines im Handel erhältlichen Harzes für den 3D-Druck, ausgewählt aus Acrylharzen, gegebenenfalls Homo- oder Copolymeren oder deren Mischungen, und Silikonharzen;
(2) Perkolieren der in Anspruch 1 definierten Zusammensetzung, die das gleiche Harz umfasst, das für den Druck im vorherigen Schritt verwendet wurde, auf das im vorherigen Schritt hergestellte Objekt.

7. Verfahren nach Anspruch 6, umfassend die folgende Vorbehandlung des Materials pflanzlichen Ursprungs:
(a) Waschen und Entfernen von Erde, gegebenenfalls 30 Minuten bei Raumtemperatur in denaturiertem Wasser, durch anschließendes Eintauchen in Tanks; 30
(b) Ultraschallbad in Wasser, gegebenenfalls für 15 Minuten; 2
(c) Ausblasen mit hohem Druck, um die Verunreinigungen vollständig zu beseitigen, gegebenenfalls in wiederholten Schritten, die etwa 10 Minuten lang durchgeführt werden;
(d) erstes Dämpfen für eine Stunde mit Wasserdampf bei 70-90 °C, um die Bakterien zu beseitigen;
(e) zweites Dämpfen bei 105 °C für 5 Stunden mit Belüftung oder bei 120° für 20 Minuten oder mit einem Wirbelschichttrockner mit Zuführ-Leitungswegen; 5
(f) Zerkleinern, gegebenenfalls mit einem Messerbrecher;
(g) erstes Vermahlen, wahlweise mit einer Hammermühle;
(h) zweites Vermahlen, gegebenenfalls mit einer Nagelmühle, um Partikeldurchmesser von etwa 0,5 mm zu erreichen; und
(i) Mikronisierung, gegebenenfalls mit einem Mikronisierer mit elliptischer Kammer oder mit einer Mühle mit gegenüberliegenden Düsen, um einen Durchmesser von unter 100 Mikron und bis zu 50 Mikron zu erreichen.

8. Erzeugnis, erhalten durch das Verfahren nach einem der Ansprüche 6 oder 7.

9. Erzeugnis nach Anspruch 8, bei dem das Organ ein Herz ist.

## Revendications

1. Utilisation d'une composition comprenant une ou plusieurs résines disponibles dans le commerce pour l'impression 3D, choisies parmi les résines acryliques, facultativement les homo- ou copolymères polyacrylates ou leurs mélanges, et les résines de silicones, mélangées à une matière d'origine végétale choisie dans les familles des Solenaceae, facultativement les pommes de terre, les aubergines, les tomates, les poivrons, et dans le genre Fragaria, facultativement les fraises, dans un procédé de préparation de modèles d'organes ou de tissus.

2. Utilisation selon la revendication 1, dans laquelle les parties de plantes, dont provient la matière végétale, sont des feuilles, des racines ou des tiges ou leurs mélanges.

3. Utilisation selon l'une quelconque des revendications 1 ou 2, dans laquelle la matière d'origine végétale provient de déchets agricoles.

4. Utilisation selon l'une quelconque des revendications 1 ou 3, dans laquelle la matière d'origine végétale se présente sous la forme de particules ayant une taille moyenne comprise entre 50 et 100 microns.

5. Utilisation selon la revendication 1, dans laquelle le procédé comprend les étapes suivantes :
1) impression d'un modèle d'organe ou de tissu en utilisant une résine disponible dans le commerce pour l'impression 3D choisie parmi les résines acryliques, facultativement les homo- ou copolymères ou leurs mélanges, et les résines de silicones ;
2) percolation sur le modèle préparé à l'étape précédente de la composition telle que définie dans la revendication 1, comprenant la même résine utilisée pour l'impression à l'étape précédente.

6. Procédé de préparation d'un objet imprimé en 3D, dans lequel l'objet imprimé en 3D est un modèle d'organe ou de tissus, comprenant les étapes suivantes :
1) impression de l'objet en utilisant une résine disponible dans le commerce pour l'impression 3D choisie parmi les résines acryliques, facultativement les homo- ou copolymères ou leurs mélanges, et les résines de silicones ;
2) percolation sur l'objet, préparé à l'étape précédente, de la composition telle que définie dans la revendication 1, comprenant la même résine utilisée pour l'impression à l'étape précédente.

7. Procédé selon la revendication 6, comprenant le prétraitement suivant de la matière d'origine végétale :
a) lavage et élimination des salissures, facultativement 30 minutes à température ambiante dans de l'eau dénaturée, par immersions successives dans des cuves ; 30
b) bain à ultrasons dans de l'eau, facultativement pendant 15 minutes ; 2
c) soufflage à haute pression, afin d'éliminer complètement les impuretés, facultativement par étapes répétées réalisées pendant environ 10 minutes ;
d) premier étuvage pendant une heure avec de la vapeur d'eau à 70-90°C, pour éliminer les bactéries ;
e) second étuvage à 105°C pendant 5 heures avec ventilation ou à 120° pendant 20 minutes ou avec un sécheur à lit fluidisé avec caténaires insufflées ; 5
f) concassage, facultativement avec un concasseur à lames ;
g) premier broyage, facultativement avec un broyeur à marteaux ;
h) second broyage, facultativement avec un broyeur à clous, pour atteindre des diamètres de particules d'environ 0,5 mm ; et
i) micronisation, facultativement avec un microniseur à chambre elliptique ou avec un broyeur 10 à jets opposés, pour atteindre moins de 100 microns et jusqu'à 50 microns.

8. Produit obtenu par le procédé selon l'une quelconque des revendications 6 ou 7.

9. Produit selon la revendication 8, l'organe étant un coeur.
